# EUROPEAN PATENT SPECIFICATION

(11) **EP 1 259 814 B1**
(45) Date of publication and mention of the grant of the patent: **12.10.2005**
(21) Application number: 01905953.4
(22) Date of filing: 21.02.2001
(51) Int. Cl.: G01N 33/574, A61P 35/00

(54) **BCMP-7 AS MARKER FOR DIAGNOSIS OF BREAST CANCER**
DIAGNOSE VON BRUSTKREBS UNTER VERWENDUNG VON BCMP-7 ALS MARKER
BCMP 7 EN TANT QUE MARQUEUR POUR LE DIAGNOSTIC DU CANCER DU SEIN

(30) Priority: 25.02.2000 GB 0004576
(43) Date of publication of application: 27.11.2002
(73) Proprietor: Oxford GlycoSciences (UK) Limited, Oxon OX14 4RY (GB)
(72) Inventor: Boyd, Robert, Simon Oxford GlycoSciences (UK) Ltd, Abingdon Oxfordshire OX14 4RY (GB); Stamps, Alasdair C. Oxford GlycoSciences (UK) Ltd, Abingdon Oxfordshire OX14 4RY (GB); Terrett, Jonathan A. Oxford GlycoSciences UK Ltd, Abingdon Oxfordshire OX14 4RY (GB); Tyson, Kerry, Louise Oxford GlycoSciences (UK) Ltd, Abingdon Oxfordshire OX14 4RY (GB)
(74) Representative: Thompson, John
(86) International application number: PCT/GB2001/000734
(87) International publication number: WO 2001/063290

(56) References cited:
- WO-A-00/53755
- WO-A-00/55629
- WO-A-98/07749
- DE-A- 19 817 557
- THOMPSON DEVON A ET AL: "hAG-2, the human homologue of the Xenopus laevis cement gland gene 4 XAG-2, is coexpressed with estrogen receptor in breast cancer cell lines." BIOCHEMICAL AND BIOPHYSICAL RESEARCH COMMUNICATIONS, vol. 251, no. 1, 9 October 1998 (1998-10-09), pages 111-116, XP001009725 ISSN: 0006-291X cited in the application -& DATABASE EMBL [Online] ID/AC: 088312, 1 November 1998 (1998-11-01) XP002169878

### SEQUENCE LISTING

<110> Oxford Glycosciences (UK) Ltd.
   Boyd, Robert Simon
   Stamps, Alasdair Craig
   Terrett, Jonathan Alexander
   Tyson, Kell Louise
<120> BCMP7
<130> NJL/P32141WO
<140> PCT/GB01/00734
   <141> 2001-02-21
<150> GB 0004576.5
   <151> 2000-02-25
<160> 8
<170> PatentIn version 3.0
<220> 1
   <211> 11
   <212> PRT
   <213> Homo sapiens
<400> 1
<210> 2
   <211> 11
   <212> PRT
   <213> Homo sapiens
<400> 2
   His Leu Ser Pro Asp Gly Gln Tyr Val Pro Arg
   1 5 10
<210> 3
   <211> 30
   <212> DNA
   <213> Artificial sequence
<220>
   <221>
   <223> primer
<400> 3
   catctcgagc agagttgcca tggagaaaat 30
<210> 4
   <211> 33
   <212> DNA
   <213> Artificial sequence
<220>
   <221>
   <223> primer
<400> 4
   catggatcct ttctttacaa ttcagtcttc agc 33
<210> 5
   <211> 20
   <212> DNA
   <213> Artificial sequence
<220>
   <221>
   <223> primer
<400> 5
   agataccaca gtcaaacctg 20
<210> 6
   <211> 20
   <212> DNA
   <213> Artificial sequence
<220>
   <221>
   <223> primer
<400> 6
   gcactcatcc aagtgatgaa                                                20
<210> 7
   <211> 543
   <212> DNA
   <213> Homo sapiens
<220>
   <221> CDS
   <222> (II)..(535)
<400> 7
   cagagttgcc atg gag aaa atc cca gtg tca gca ttc ttg ctc ctt gtg       49
              Met Glu Lys Ile Pro Val Ser Ala Phe Leu Leu Leu Val
              1               5                   10
   gcc ctc tcc tac act ctg gcc aga gat acc aca gtc aaa cct gga gcc      97
   Ala Leu Ser Tyr Thr Leu Ala Arg Asp Thr Thr Val Lys Pro Gly Ala
       15                  20                  25
   aaa aag gac aca aag gac tct cga ccc aaa ctg ccc cag acc ctc tcc     145
   Lys Lys Asp Thr Lys Asp Ser Arg Pro Lys Leu Pro Gln Thr Leu Ser
   30                  35                  40                  45
   aga ggt tgg ggt gac caa ctc atc tgg act cag aca tat gaa gaa gct     193
   Arg Gly Trp Gly Asp Gln Leu Ile Trp Thr Gln Thr Tyr Glu Glu Ala
                   50                  55                  60
   cta tat aaa tcc aag aca agc aac aaa ccc ttg atg att att cat cac        241
   Leu Tyr Lys Ser Lys Thr Ser Asn Lys Pro Leu Met Ile Ile His His
               65                  70                  75
   ttg gat gag tgc cca cac agt caa gct tta aag aaa gtg ttt gct gaa        289
   Leu Asp Glu Cys Pro His Ser Gln Ala Leu Lys Lys Val Phe Ala Glu
           80                  85                  90
   aat aaa gaa atc cag aaa ttg gca gag cag ttt gtc ctc ctc aat ctg        337
   Asn Lys Glu Ile Gln Lys Leu Ala Glu Gln Phe Val Leu Leu Asn Leu
       95                  100                 105
   gtt tat gaa aca act gac aaa cac ctt tct cct gat ggc cag tat gtc        385
   Val Tyr Glu Thr Thr Asp Lys His Leu Ser Pro Asp Gly Gln Tyr Val
   110                 115                 120                 125
   ccc agg att atg ttt gtt gac cca tct ctg aca gtt aga gcc gat atc        433
   Pro Arg Ile Met Phe Val Asp Pro Ser Leu Thr Val Arg Ala Asp Ile
                   130                 135                 140
   act gga aga tat tca aac cgt ctc tat gct tac gaa cct gca gat aca        481
   Thr Gly Arg Tyr Ser Asn Arg Leu Tyr Ala Tyr Glu Pro Ala Asp Thr
               145                 150                 155
   gct ctg ttg ctt gac aac atg aag aaa gct ctc aag ttg ctg aag act        529
   Ala Leu Leu Leu Asp Asn Met Lys Lys Ala Leu Lys Leu Leu Lys Thr
           160                 165                 170
   gaa ttg taaagaaa                                                       543
   Glu Leu
       175
<210> 8
   <211> 175
   <212> PRT
   <213> Homo sapiens
<400> 8
   Met Glu Lys Ile Pro Val Ser Ala Phe Leu Leu Leu Val Ala Leu Ser
   1               5                   10                  15
   Tyr Thr Leu Ala Arg Asp Thr Thr Val Lys Pro Gly Ala Lys Lys Asp
               20                  25                  30
   Thr Lys Asp Ser Arg Pro Lys Leu Pro Gln Thr Leu Ser Arg Gly Trp
           35                  40                  45
   Gly Asp Gln Leu Ile Trp Thr Gln Thr Tyr Glu Glu Ala Leu Tyr Lys
       50                  55                  60
   Ser Lys Thr Ser Asn Lys Pro Leu Met Ile Ile His His Leu Asp Glu
   65                  70                  75                  80
   Cys Pro His Ser Gln Ala Leu Lys Lys Val Phe Ala Glu Asn Lys Glu
                   85                  90                  95
   Ile Gln Lys Leu Ala Glu Gln Phe Val Leu Leu Asn Leu Val Tyr Glu
               100                 105                 110
   Thr Thr Asp Lys His Leu Ser Pro Asp Gly Gln Tyr Val Pro Arg Ile
           115                 120                 125
   Met Phe Val Asp Pro Ser Leu Thr Val Arg Ala Asp Ile Thr Gly Arg
       130                 135                 140
   Tyr Ser Asn Arg Leu Tyr Ala Tyr Glu Pro Ala Asp Thr Ala Leu Leu
   145                 150                 155                 160
   Leu Asp Asn Met Lys Lys Ala Leu Lys Leu Leu Lys Thr Glu Leu
                   165                 170                 175

## Description

The present invention relates to the diagnosis, prophylaxis and treatment of breast cancer, and in particular to the use of a protein which has been isolated from breast cancer cell line membrane preparations, compositions comprising the protein, including vaccines and antibodies which are immunospecific for the protein, in such diagnosis, prophylaxis and treatment.

Breast cancer is the most frequently diagnosed cancer in women. The implementation of screening programs for the early detection of breast cancer, and the advent of anticancer treatments, such as chemotherapy, radiotherapy and anti-oestrogen therapies, to augment surgical resection have improved the survival of breast cancer patients.

However, some breast tumours become refractory to such treatments, as the cancer cells develop resistance to chemotherapy drugs or lose their hormone sensitivity, leading to recurrent or metastatic disease which is often incurable. More recently, attention has focussed on the development of immunological therapies (Green, *et al*, *Cancer Treat. Rev.* **26,** 269-286 (2000); Davis, *Immunol. Cell Biol.* **78**, 179-195 (2000); Knuth *et al*, *Cancer Chemother Pharmacol.* **46,** S46-51 (2000); Shiku, *et al*, *Cancer Chemother. Pharmacol*. **46**, S77-82 (2000); Saffran, *et al, Cancer Metastasis Rev.* **18**, 437-449 (1999)), such as cancer vaccines and monoclonal antibodies (mAbs), as a means of initiating and targeting a host immune response against tumour cells. In 1998 the FDA approved the use of Herceptin (Stebbing, *et al*, *Cancer Treat. Rev.* **26**, 287-290 (2000); Dillman, *Cancer Biother. Radiopharm.* **14**, 5-10 (1999); Miller & Sledge, *Invest. New Drugs* **17**, 417-427 (1999)), a mAb that recognises the erbB2/HER2-neu receptor protein, as a treatment for metastatic breast cancer. In combination with chemotherapy, Herceptin has been shown to prolong the time to disease progression, when compared to patients receiving chemotherapy alone (Baselga, *et al*, *Cancer Res.* **58,** 2825-2831 (1998)). However, Herceptin is only effective in treating the 10-20% of patients whose tumours over-express the erbB2 protein. Thus, the identification of other suitable targets or antigens for immunotherapy of breast cancer has become increasingly important.

An ideal protein target for cancer immunotherapy should have a restricted expression profile in normal tissues and be over-expressed in tumours, such that the immune response will be targeted to tumour cells and not against other organs. In addition, the protein target should be exposed on the cell surface, where it will be accessible to therapeutic agents. Tumour antigens have been identified for a number of cancer types, by using techniques such as differential screening of cDNA (Hubert, *et al*, *Proc. Natl. Acad, Sci. USA* **96,** 14523-14528 (1999); Lucas, *et al*, *Int. J. Cancer* **87**, 55-60 (2000)), and the purification of cell-surface antigens that are recognised by tumour-specific antibodies (Catimel, *el al*, *J. Biol. Chem*. **271,** 25664-25670 (1996)). As an alternative approach to identifying breast tumour antigens, we have used proteomics to characterise the complement of proteins in cell membranes isolated from the breast cancer cell line T-47D (ATCC:HTB-133). In this way, we have identified a protein, designated BCMP 7, which shows elevated expression in some breast cancer lines, suggesting that it may be a suitable target for cancer therapy and diagnosis.

BCMP 7 is known. For example, WO98/07749 discloses novel human growth factors. These include a sequence, identified as huXAG-1, which corresponds to BCMP 7 discussed herein. WO99/53040 discloses a large number of sequences derived from an EST database, including sequences, identified as sequences ID 265 and 288, which correspond to BCMP 7 discussed herein. These sequences are indicated as being more highly expressed in ovarian cancer tissue. WO99/55858 discloses a large number of sequences derived from an EST database. These include sequences, identified as sequences ID 8 and 181, which correspond to BCMP 7 discussed herein, which are indicated as being more highly expressed in pancreas cancer tissue. WO09841627 discloses a number of sequences and in particular Seq ID Nos 1 and 2 which correspond to BCMP 7 discussed herein. WO98211217 also discloses sequences (Seq ID Nos 9 and 27) that correspond to BCMP 7 discussed herein, which were obtained from stomach cancer. WO0053755 discloses a sequence (PRO 1030) which corresponds to BCMP 7 described herein. Gene copy amplication was used to quantify the relevance of the protein to a set of tumours. Of these, the number of gene copies are increased in primary lung tumour and primary colon tumour. Sequences corresponding to BCMP 7 are also present in WO9940189. BCMP 7 has also been shown to be co-expressed with estrogen receptor in breast cancer cell lines (Thompson & Weigel, 1998, *Biochem. Biophys. Res. Commun.* **251,** 111-116).

However; these prior art documents do not reveal the involvement of BCMP 7 in breast cancer, and/or do not identify BCMP 7 as being localised to the peripheral membrane and therefore useful in an immunotherapeutic approach to breast cancer.

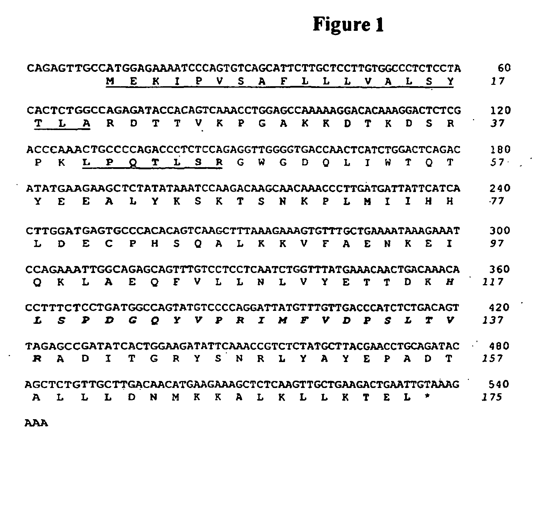

Thus, in a first aspect, the present invention provides a method of screening for and/or diagnosis of breast cancer in a subject, which method comprises the step of detecting and/or quantifying the amount of a polypeptide in a biological sample obtained from said subject, wherein the polypeptide:
a) comprises or consists of the amino acid sequence shown in figure 1;
b) is a derivative having one or more amino acid substitutions, deletions or insertions relative to the amino acid sequence shown in figure 1; or
c) is a fragment of a polypeptide as defined in a) or b) above, which is at least ten amino acids long.

In a second aspect, the present invention provides a method for the prophylaxis and/or treatment of breast cancer in a subject, which comprises administering to said subject a therapeutically effective amount of at least one polypeptide as defined in the first aspect of the invention.

In a third aspect, the present invention provides the use of at least one polypeptide as defined in the first aspect of the invention in the preparation of a medicament for use in the prophylaxis and/or treatment of breast cancer.

As used herein, breast tissue refers to the breast itself, as well as the tissue adjacent and/or within the strata underlying the breast. The subject may be a mammal and is preferably a human, although monkeys, apes, cats, dogs, cows, horses and rabbits are within the scope of the present invention.

In the second and third aspects, the polypeptides or fragments thereof may be provided in isolated or recombinant form, and may be fused to other moieties. In particular, fusions of the polypeptides or fragments thereof with localisation-reporter proteins such as the Green Fluorescent Protein (U.S. Patent Nos. 5,625,048, 5,777,079, 6,054,321 and 5,804,387) or the DsRed fluorescent protein (Matz,*et al* (1999) *Nature Biotech*. 17:969-973) are specifically contemplated. The polypeptides or fragments thereof may be provided in substantially pure form, that is to say free, to a substantial extent, from other proteins. Thus, a polypeptide may be provided in a composition in which it is the predominant component present (i.e. it is present at a level of at least 50%; preferably at least 75%, at least 90%, or at least 95%; when determined on a weight/weight basis excluding solvents or carriers).

In order to more fully appreciate the present invention, polypeptides within the scope of a)-c) above will now be discussed in greater detail.

### Polypeptides within the scope of a)

A polypeptide within the scope of a), may consist of the particular amino acid sequence given in Figure 1 or may have an additional N-terminal and/or an additional C-terminal amino acid sequence relative to the sequence given in Figure 1.

Additional N-terminal or C-terminal sequences may be provided for various reasons. Techniques for providing such additional sequences are well known in the art.
Additional sequences may be provided in order to alter the characteristics of a particular polypeptide. This can be useful in improving expression or regulation of expression in particular expression systems. For example, an additional sequence may provide some protection against proteolytic cleavage. This has been done for the hormone Somatostatin by fusing it at its N-terminus to part of the β galactosidase enzyme (Italcwa *et al., Science* **198**: 105-63 (1977)).

Additional sequences can also be useful in altering the properties of a polypeptide to aid in identification or purification. For example, a fusion protein may be provided in which a polypeptide is linked to a moiety capable of being isolated by affinity chromatography. The moiety may be an antigen or an epitope and the affinity column may comprise immobilised antibodies or immobilised antibody fragments which bind to said antigen or epitope (desirably with a high degree of specificity). The fusion protein can usually be eluted from the column by addition of an appropriate buffer.

Additional N-terminal or C-terminal sequences may, however, be present simply as a result of a particular technique used to obtain a polypeptide and need not provide any particular advantageous characteristic to the polypeptide. Such polypeptides are within the scope of the present invention.

Whatever additional N-terminal or C-terminal sequence is present, it is preferred that the resultant polypeptide should exhibit the immunological or biological activity of the polypeptide having the amino acid sequence shown in Figure 1.

### Polypeptides within the scope of b)

Turning now to the polypeptides defined in b) above, it will be appreciated by the person skilled in the art that these polypeptides are variants of the polypeptide given in a) above.

Such variants preferably exhibit the immunological or biological activity of the polypeptide having the amino acid sequence shown in Figure 1.

Alterations in the amino acid sequence of a protein can occur which do not affect the function of a protein. These include amino acid deletions, insertions and substitutions and can result from alternative splicing and/or the presence of multiple translation start sites and stop sites. Polymorphisms may arise as a result of the infidelity of the translation process. Thus changes in amino acid sequence may be tolerated which do not affect the protein's biological or immunological function.

The skilled person will appreciate that various changes can often be made to the amino acid sequence of a polypeptide which has a particular activity to produce variants (sometimes known as "muteins") having at least a proportion of said activity, and preferably having a substantial proportion of said activity. Such variants of the polypeptides described in a) above are within the scope of the present invention and are discussed in greater detail below. They include allelic and non-allelic variants.

An example of a variant of the present invention is a polypeptide as defined in a) above, apart from the substitution of one or more amino acids with one or more other amino acids. The skilled person is aware that various amino acids have similar properties. One or more such amino acids of a polypeptide can often be substituted by one or more other such amino acids without eliminating a desired activity of that polypeptide.

Thus, the amino acids glycine, alanine, valine, leucine and isoleucine can often be substituted for one another (amino acids having aliphatic side chains). Of these possible substitutions, it is preferred that glycine and alanine are used to substitute for one another (since they have relatively short side chains) and that valine, leucine and isoleucine are used to substitute for one another (since they have larger aliphatic side chains which are hydrophobic).

Other amino acids which can often be substituted for one another include:
- phenylalanine, tyrosine and tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains); and
- cysteine and methionine (amino acids having sulphur-containing side chains).

Substitutions of this nature are often referred to as "conservative" or "semi-conservative" amino acid substitutions.

Amino acid deletions or insertions may also be made relative to the amino acid sequence given in a) above. Thus, for example, amino acids which do not have a substantial effect on the biological and/or immunological activity of the polypeptide, or at least which do not eliminate such activity, may be deleted. Such deletions can be advantageous since the overall length and the molecular weight of a polypeptide can be reduced whilst still retaining activity. This can enable the amount of polypeptide required for a particular purpose to be reduced - for example, dosage levels can be reduced.

Amino acid insertions relative to the sequence given in a) above can also be made. This may be done to alter the properties of a polypeptide used in the present invention (e.g. to assist in identification, purification or expression, as explained above in relation to fusion proteins).

Amino acid changes relative to the sequence given in a) above can be made using any suitable technique e.g. by using site-directed mutagenesis (Hutchinson *et al*., 1978, *J. Biol. Chem*. **253**:6551).

It should be appreciated that amino acid substitutions or insertions within the scope of the present invention can be made using naturally occurring or non-naturally occurring amino acids. Whether or not natural or synthetic amino acids are used, it is preferred that only L- amino acids are present.

Whatever amino acid changes are made (whether by means of substitution, insertion or deletion), preferred polypeptides of the present invention have at least 50% sequence identity with a polypeptide as defined in a) above, more preferably the degree of sequence identity is at least 75%. Sequence identities of at least 90% or at least 95% are most preferred.

The percent identity of two amino acid sequences or of two nucleic acid sequences is determined by aligning the sequences for optimal comparison purposes (*e.g*., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared (*i.e*., % identity = # of identical positions/total # of positions x 100).

The determination of percent identity between two sequences can be accomplished using a mathematical algorithm known to those of skill in the art. An example of a mathematical algorithm for comparing two sequences is the algorithm of Karlin and Altschul (1990) *Proc. Natl. Acad. Sci. USA* 87:2264-2268, modified as in Karlin and Altschul (1993) *Proc. Natl. Acad. Sci. USA* 90:5873-5877. The NBLAST and XBLAST programs of Altschul, et al. (1990) *J. Mol. Biol*. 215:403-410 have incorporated such an algorithm. BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilised as described in Altschul et al. (1997) *Nucleic Acids Res.* 25:3389-3402. Alternatively, PSI-Blast can be used to perform an iterated search which detects distant relationships between molecules (*Id*.). When utilising BLAST, Gapped BLAST, and PSI-Blast programs, the default parameters of the respective programs (e.g., XBLAST and NBLAST) can be used. *See* http://www.ncbi.nlm.nih.gov.

Another example of a mathematical algorithm utilised for the comparison of sequences is the algorithm of Myers & Miller, CABIOS (1989). The ALIGN program (version 2.0) which is part of the CGC sequence alignment software package has incorporated such an algorithm. Other algorithms for sequence analysis known in the art include ADVANCE and ADAM as described in Torellis & Robotti (1994) *Comput. Appl. Biosci., 10* :3-5; and FASTA described in Pearson & Lipman (1988) *Proc. Natl. Acad. Sci. 85:2444-8.* Within FASTA, ktup is a control option that sets the sensitivity and speed of the search.

Where high degrees of sequence identity are present there will be relatively few differences in amino acid sequence. Thus for example they may be less than 20, less than 10, or even less than 5 differences.

### Polypeptides within the scope of c)

As discussed *supra,* it is often advantageous to reduce the length of a polypeptide, provided that the resultant reduced length polypeptide still has a desired activity or can give rise to useful antibodies. Feature c) of the present invention therefore covers fragments of polypeptides a) or b) above.

The skilled person can determine whether or not a particular fragment has activity using the techniques disclosed above. Preferred fragments are at least 10 amino acids long. They may be at least 20, at least 50 or at least 100 amino acids long.

A polypeptide as defined herein may be useful as antigenic material, and may be used in the production of vaccines for treatment or prophylaxis of breast cancer. Such material can be "antigenic" and/or "immunogenic". Generally, "antigenic" is taken to mean that the protein is capable of being used to raise antibodies or indeed is capable of inducing an antibody response in a subject. "Immunogenic" is taken to mean that the protein is capable of eliciting a protective immune response in a subject. Thus, in the latter case, the protein may be capable of not only generating an antibody response but, in addition, non-antibody based immune responses.

It is well known that is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments of the present invention may include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue may be that the fragment retains the antigenic/immunogenic properties of the protein from which it is derived.

Homologues, derivatives and fragments may possess at least a degree of the antigenicity/immunogenicity of the protein or polypeptide from which they are derived.

In a further aspect, the present invention provides the use of a polypeptide as defined herein in the production of a medicament for the treatment or prophylaxis of breast cancer, wherein the medicament is a vaccine. The vaccine optionally comprises one or more suitable adjuvants. Examples of adjuvants well-known in the art include inorganic gels, such as aluminium hydroxide, and water-in-oil emulsions, such as incomplete Freund's adjuvant. Other useful adjuvants will be well known to the skilled person.

In yet further aspects, the present invention provides:
(a) the use of a polypeptide as defined herein in the preparation of an immunogenic composition, preferably a vaccine;
(b) the use of such an immunogenic composition in inducing an immune response in a subject; and
(c) a method for the treatment or prophylaxis of breast cancer in a subject, or of vaccinating a subject against breast cancer which comprises the step of administering to the subject an effective amount of a polypeptide as defined herein, preferably as a vaccine.

As will be discussed below, the polypeptides used in the present invention will find use in an immunotherapeutic approach to breast cancer. The skilled person will appreciate that for the preparation of one or more such polypeptides, the preferred approach will be based on recombinant DNA techniques. In addition, nucleic acid molecules encoding the polypeptides or fragments thereof may be used in their own right. Thus, in a fourth aspect, the invention provides a method of screening for and/or diagnosis of breast cancer in a subject, which method comprises the step of detecting and/or quantifying the amount of a nucleic acid in a biological sample obtained from said subject, wherein the nucleic acid molecule:
a) comprises or consists of the DNA sequence shown in Figure 1 or its RNA equivalent;
b) has a sequence which is complementary to the sequences of a);
c) has a sequence which codes for the same polypeptide as the sequences of

a) or b);
d) has a sequence which shows substantial identity with any of those of a), b) and c); or
e) a sequence which codes for a derivative or fragment of an amino acid molecule shown in Figure 1.

In a fifth aspect, the present invention provides a method for the prophylaxis and/or treatment of breast cancer in a subject, which comprises administering to said subject a therapeutically effective amount of at least one nucleic acid as defined in the fourth aspect of the invention.

In a sixth aspect, the present invention provides the use of at least one nucleic acid as defined in the fourth aspect of the invention in the preparation of a medicament for use in the prophylaxis and/or treatment of breast cancer.

These nucleic acid molecules are now discussed in greater detail.

It is preferred if sequences which show substantial identity with any of those of a), b) and c) have e.g. at least 50%, at least 75% or at least 90% or 95% sequence identity.

The polypeptides used in the present invention can be coded for by a large variety of nucleic acid molecules, taking into account the well known degeneracy of the genetic code. All of these molecules are within the scope of the present invention. They can be inserted into vectors and cloned to provide large amounts of DNA or RNA for further study. Suitable vectors may be introduced into host cells to enable the expression of polypeptides used in the present invention using techniques known to the person skilled in the art.

The term 'RNA equivalent' when used above indicates that a given RNA molecule has a sequence which is complementary to that of a given DNA molecule, allowing for the fact that in RNA 'U' replaces 'T' in the genetic code. The nucleic acid molecule may be in isolated, recombinant or chemically synthetic form.

Techniques for cloning, expressing and purifying proteins and polypeptides are well known to the skilled person. DNA constructs can readily be generated using methods well known in the art. These techniques are disclosed, for example in J. Sambrook *et* *al, Molecular Cloning 2*^{*nd*} *Edition,* Cold Spring Harbour Laboratory Press (1989); in Old & Primrose *Principles of Gene Manipulation* 5th Edition, Blackwell Scientific Publications (1994); and in Stryer, *Biochemistry* 4th Edition, W H Freeman and Company (1995). Modifications of DNA constructs and the proteins expressed such as the addition of promoters, enhancers, signal sequences, leader sequences, translation start and stop signals and DNA stability controlling regions, or the addition of fusion partners may then be facilitated.

Normally the DNA construct will be inserted into a vector, which may be of phage or plasmid origin. Expression of the protein is achieved by the transformation or transfection of the vector into a host cell which may be of eukaryotic or prokaryotic origin. Such vectors and suitable host cells form aspects of the present invention.

The nucleotide sequences of the present invention, including DNA and RNA, and comprising a sequence encoding a polypeptide as defined herein (or a fragment, homologue or analogue thereof), may be synthesised using methods known in the art, such as using conventional chemical approaches or polymerase chain reaction (PCR) amplification. The nucleotide sequences of the present invention also permit the identification and cloning of the gene encoding a polypeptide as defined herein from any species, for instance by screening cDNA libraries, genomic libraries or expression libraries.

Knowledge of the nucleic acid structure can be used to raise antibodies and for gene therapy. Techniques for this are well-known by those skilled in the art, as discussed in more detail herein.

By using appropriate expression systems, polypeptides of the present invention may be expressed in glycosylated or non-glycosylated form. Non-glycosylated forms can be produced by expression in prokaryotic hosts, such as *E. coli.*

Polypeptides comprising N-terminal methionine may be produced using certain expression systems, whilst in others the mature polypeptide will lack this residue.

Preferred techniques for cloning, expressing and purifying a polypeptide used in the present invention are summarised below:

Polypeptides may be prepared natively or under denaturing conditions and then subsequently refolded. Baculoviral expression vectors include secretory plasmids (such as pACGP67 from Pharmingen), which may have an epitope tag sequence cloned in frame (e.g. myc, V5 or His) to aid detection and allow for subsequent purification of the protein. Mammalian expression vectors may include pCDNA3 and pSecTag (both Invitrogen), and pREP9 and pCEP4 (invitrogen). *E. coli* systems include the pBad series (His tagged - Invitrogen) or pGex series (Pharamacia).

In addition to nucleic acid molecules coding for polypeptides used in the present invention, referred to herein as "coding" nucleic acid molecules, the present invention also includes nucleic acid molecules complementary thereto. Thus, for example, both strands of a double stranded nucleic acid molecule are included within the scope of the present invention (whether or not they are associated with one another). Also included are mRNA molecules and complementary DNA Molecules (e.g. cDNA molecules).

Nucleic acid molecules which can hybridise to any of the nucleic acid molecules discussed above are also covered by the present invention. Such nucleic acid molecules are referred to herein as "hybridising" nucleic acid molecules. Hybridising nucleic acid molecules can be useful as probes or primers, for example.

Desirably such hybridising molecules are at least 10 nucleotides in length and preferably are at least 25 or at least 50 nucleotides in length. The hybridising nucleic acid molecules preferably hybridise to nucleic acids within the scope of (a), (b), (c), (d) or (e) above specifically.

Desirably the hybridising molecules will hybridise to such molecules under stringent hybridisation conditions. One example of stringent hybridisation conditions is where attempted hybridisation is carried out at a temperature of from about 35°C to about 65°C using a salt solution which is about 0.9 molar. However, the skilled person will be able to vary such conditions as appropriate in order to take into account variables such as probe length, base composition, type of ions present, etc. For a high degree of selectivity, relatively stringent conditions are used to form the duplexes, such as low salt or high temperature conditions. As used herein, "highly stringent conditions" means hybridisation to filter-bound DNA in 0.5 M NaHPO₄, 7% sodium dodecyl sulphate (SDS), 1 mM EDTA at 65 °C, and washing in 0.1xSSC/0.1% SDS at 68 °C (Ausubel F.M. et al., eds., 1989, Current Protocols in Molecular Biology, Vol. I, Green Publishing Associates, Inc., and John Wiley & Sons, Inc., New York, at p. 2.10.3) For some applications, less stringent conditions for duplex formation are required. As used herein "moderately stringent conditions" means washing in 0.2xSSC/0.1% SDS at 42 °C (Ausubel et al., 1989, *supra*). Hybridisation conditions can also be rendered more stringent by the addition of increasing amounts of formamide, to destabilise the hybrid duplex. Thus, particular hybridisation conditions can be readily manipulated, and will generally be chosen depending on the desired results. In general, convenient hybridisation temperatures in the presence of 50% formamide are: 42 °C for a probe which is 95 to 100% identical to the fragment of a gene encoding a polypeptide as defined herein, 37°C for 90 to 95% identity and 32 °C for 70 to 90% identity. In the preparation of genomic libraries, DNA fragments are generated, some of which will encode parts or the whole of a polypeptide as defined herein. The DNA may be cleaved at specific sites using various restriction enzymes. Alternatively, one may use DNAse in the presence of manganese to fragment the DNA, or the DNA can be physically sheared, as for example, by sonication. The DNA fragments can then be separated according to size by standard techniques, including but not limited to agarose and polyacrylamide gel electrophoresis, column chromatography and sucrose gradient centrifugation. The DNA fragments can then be inserted into suitable vectors, including but not limited to plasmids, cosmids, bacteriophages lambda or T₄, and yeast artificial chromosomes (YACs). (See, for example, Sambrook et al., 1989, *Molecular* *Cloning,* A Laboratory Manual, 1D Ed., Cold Spring Harbor Laboratory Press, Cold Spring Harbor, New York; Glover, D.M. (ed.), 1985, *DNA Cloning: A Practical Approach,* MRL Press, Ltd., Oxford, U.K. Vol. I, II; Ausubel F.M. et al., eds., 1989, Current Protocols in Molecular Biology, Vol. I, Green Publishing Associates, Inc., and John Wiley & sons, Inc., New York). The genomic library may be screened by nucleic acid hybridisation to labelled probe (Benton & Davis, 1977, *Science* **196**:180; Grunstein & Hogness, 1975, *Proc. Natl. Acad. Sci. U.S.A.* **72**:3961).

Manipulation of the DNA encoding a protein is a particularly powerful technique for both modifying proteins and for generating large quantities of protein for purification purposes. This may involve the use of PCR techniques to amplify a desired nucleic acid sequence. Thus the sequence data provided herein can be used to design primers for use in PCR so that a desired sequence can be targeted and then amplified to a high degree.

Typically primers will be at least five nucleotides long and will generally be at least ten nucleotides long (e.g. fifteen to twenty-five nucleotides long). In some cases, primers of at least thirty or at least thirty-five nucleotides in length may be used.

As a further alternative chemical synthesis may be used. This may be automated. Relatively short sequences may be chemically synthesised and ligated together to provide a longer sequence.

In addition to being used as primers and/or probes, hybridising nucleic acid molecules of the present invention can be used as anti-sense molecules to alter the expression of substances of the present invention by binding to complementary nucleic acid molecules. This technique can be used in anti-sense therapy.

In a specific embodiment, expression of a polypeptide as defined herein is inhibited by use of antisense nucleic acids. The present invention provides the therapeutic or prophylactic use of nucleic acids comprising at least six nucleotides that are antisense to a gene or cDNA encoding a polypeptide as defined herein or a portion thereof. As used herein, an "antisense" nucleic acid refers to a nucleic acid capable of hybridising by virtue of some sequence complementarity to a portion of an RNA (preferably mRNA) encoding a polypeptide as defined herein. The antisense nucleic acid may be complementary to a coding and/or non-coding region of a mRNA encoding such a polypeptide. Such antisense nucleic acids have utility as compounds that inhibit expression, and can be used in the treatment or prevention of breast cancer.

A hybridising nucleic acid molecule of the present invention may have a high degree of sequence identity along its length with a nucleic acid molecule within the scope of (a)-(e) above (e.g. at least 50%, at least 75% or at least 90% or 95% sequence identity). As will be appreciated by the skilled person, the higher the sequence identity a given single stranded nucleic acid molecule has with another nucleic acid molecule, the greater the likelihood that it will hybridise to a nucleic acid molecule which is complementary to that other nucleic acid molecule under appropriate conditions.

In view of the foregoing description the skilled person will appreciate that a large number of nucleic acids are within the scope of the present invention. Unless the context indicates otherwise, nucleic acid molecules of the present invention may have one or more of the following characteristics:
1) they may be DNA or RNA;
2) they may be single or double stranded;
3) they may be provided in recombinant form, e.g. covalently linked to a 5' and/or a 3' flanking sequence to provide a molecule which does not occur in nature;
4) they may be provided without 5' and/or 3' flanking sequences which normally occur in nature;
5) they may be provided in substantially pure form. Thus they may be provided in a form which is substantially free from contaminating proteins and/or from other nucleic acids; and
6) they may be provided with introns or without introns (e.g. as cDNA).

If desired, a gene encoding a polypeptide as defined herein, a related gene, or related nucleic acid sequences or subsequences, including complementary sequences, can also be used in hybridisation assays. A nucleotide encoding a polypeptide as defined herein, or subsequences thereof comprising at least 8 nucleotides, can be used as a hybridisation probe. Hybridisation assays can be used for detection, prognosis, diagnosis, or monitoring of conditions, disorders, or disease states, associated with aberrant expression of genes encoding a polypeptide as defined herein, or for differential diagnosis of patients with signs or symptoms suggestive of breast cancer. In particular, such a hybridisation assay can be carried out by a method comprising contacting a patient sample containing nucleic acid with a nucleic acid probe capable of hybridising to a DNA or RNA that encodes a polypeptide as defined herein, under conditions such that hybridisation can occur, and detecting or measuring any resulting hybridisation. Nucleotides can be used for therapy of patients having breast cancer, as described below.

In another embodiment, a preparation of oligonucleotides comprising 10 or more consecutive nucleotides complementary to a nucleotide sequence encoding a polypeptide as defined herein or fragment thereof for use as vaccines for the treatment of breast cancer. Such preparations may include adjuvants or other vehicles.

In a specific embodiment, nucleic acids comprising a sequence encoding a polypeptide as defined herein or functional derivative thereof, are administered to promote polypeptide function by way of gene therapy. Gene therapy refers to administration to a subject of an expressed or expressible nucleic acid. In this embodiment, the nucleic acid produces its encoded protein that mediates a therapeutic effect by promoting polypeptide function. Any of the methods for gene therapy available in the art can be used according to the present invention.

In a preferred aspect, the compound comprises a nucleic acid as defined herein, such as a nucleic acid encoding a polypeptide as defined herein or fragment or chimeric protein thereof, said nucleic acid being part of an expression vector that expresses a polypeptide as defined herein or fragment or chimeric protein thereof in a suitable host. In particular, such a nucleic acid has a promoter operably linked to the polypeptide coding region, said promoter being inducible or constitutive (and, optionally, tissue-specific). In another particular embodiment, a nucleic acid molecule is used in which the coding sequences and any other desired sequences are flanked by regions that promote homologous recombination at a desired site in the genome, thus providing for intrachromosomal expression of the nucleic acid (Koller & Smithies, 1989, *Proc. Natl. Acad. Sci. USA* **86**:8932-8935; Zijlstra *et al*., 1989, *Nature* **342**:435-438).

Delivery of the nucleic acid into a patient may be direct, in which case the patient is directly exposed to the nucleic acid or nucleic acid-carrying vector, this approach is known as *in vivo* gene therapy. Alternatively, delivery of the nucleic acid into the patient may be indirect, in which case cells are first transformed with the nucleic acid *in vitro* and then transplanted into the patient; this approach is known as *ex vivo* gene therapy.

A convenient means for detecting/quantifying the polypeptides used in the present invention involves the use of antibodies. Thus, the polypeptides used in the invention also find use in raising antibodies.

In a seventh aspect, the present invention provides the use of an antibody which binds to at least one polypeptide as defined in the first aspect of the invention for screening for and/or diagnosis of breast cancer in a subject. Preferably, the antibody is used for detecting and/or quantifying the amount of a polypeptide as defined in the first aspect of the invention in a biological sample obtained from said subject.

In one embodiment, binding of antibody in tissue sections can be used to detect aberrant polypeptide localisation or an aberrant level of polypeptide. In a specific embodiment, antibody to a polypeptide as defined herein can be used to assay a patient tissue (*e*.*g*., a breast biopsy) for the level of the polypeptide where an aberrant level of polypeptide is indicative of breast cancer. As used herein, an "aberrant level" means a level that is increased or decreased compared with the level in a subject free from breast cancer or a reference level. If desired, the comparison can be performed with a matched sample from the same subject, taken from a portion of the body not affected by breast cancer.

Suitable immunoassays include, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

In an eighth aspect, the present invention provides a method for the prophylaxis and/or treatment of breast cancer in a subject, which comprises administering to said subject a therapeutically effective amount of an antibody which binds to at least one polypeptide as defined in the first aspect of the invention.

In a ninth aspect, the present invention provides the use of an antibody which binds to at least one polypeptide as defined in the first aspect of the invention in the preparation of a medicament for use in the prophylaxis and/or treatment of breast cancer.

Preferred antibodies bind specifically to polypeptides of the present invention so that they can be used to purify and/or inhibit the activity of such polypeptides. The antibodies may be monoclonal or polyclonal.

Thus, the polypeptide used in the invention, its fragments or other derivatives, or analogues thereof, may be used as an immunogen to generate antibodies which immunospecifically bind such an immunogen. Antibodies of the invention include, but are not limited to polyclonal, monoclonal, bispecific, humanised or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically-active portions of immunoglobulin molecules, i.e., molecules that contain an antigen binding site that specifically binds an antigen. The immunoglobulin molecules of the invention can be of any class (e.g., IgG, IgE, IgM, IgD and IgA) or subclass of immunoglobulin molecule.

In the production of antibodies, screening for the desired antibody can be accomplished by techniques known in the art, e.g. ELISA (enzyme-linked immunosorbent assay). For example, to select antibodies which recognise a specific domain of a polypeptide used in the invention, one may assay generated hybridomas for a product which binds to a polypeptide fragment containing such domain. For selection of an antibody that specifically binds a first polypeptide homologue but which does not specifically bind to (or binds less avidly to) a second polypeptide homologue, one can select on the basis of positive binding to the first polypeptide homologue and a lack of binding to (or reduced binding to) the second polypeptide homologue.

For preparation of monoclonal antibodies (mAbs) directed toward a polypeptide used in the invention, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor *et al*., 1983, *Immunology Today* **4**:72), and the EBV-hybridoma technique to produce human monoclonal antibodies (Cole *et al.,* 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof.

The hybridoma producing the mAbs used in the invention may be cultivated *in vitro* or *in vivo.* In an additional embodiment of the invention, monoclonal antibodies can be produced in germ-free animals utilising known technology (PCT/US90/02545).

The monoclonal antibodies include but are not limited to human monoclonal antibodies and chimeric monoclonal antibodies (e.g., human-mouse chimeras). A chimeric antibody is a molecule in which different portions are derived from different animal species, such as those having a human immunoglobulin constant region and a variable region derived from a murine mAb. (See, e.g., U.S. Patent No. 4,816,567; and U.S. Patent No. 4,816397) Humanised antibodies are antibody molecules from non-human species having one or more complementarity determining regions (CDRs) from the non-human species and a framework region from a human immunoglobulin molecule. (See, e.g., U.S. Patent No. 5,585,089).

Chimeric and humanised monoclonal antibodies can be produced by recombinant DNA techniques known in the art, for example using methods described in WO 87/02671; EP-A-184,187; EP-A-171,496; EP-A-173,494; WO 86/01533; U.S. Patent No. 4,816,567; EP-A-125,023; Better *et al.,* 1988, *Science* **240**:1041-1043; Liu *et al.,* 1987, *Proc. Natl. Acad. Sci. USA* **84**:3439-3443; Liu *et al.,* 1987, *J. Immunol*. **139**:3521-3526; Sun *et al*., 1987, *Proc. Natl. Acad. Sci. USA* **84**:214-218; Nishimura *et al*., 1987, *Canc. Res.* **47**:999-1005; Wood *et al.,* 1985, *Nature* **314**:446-449; Shaw *et al*., 1988, *J. Natl. Cancer Inst*. **80**:1553-1559; Morrison, 1985, *Science* **229**:1202-1207; Oi *et al*., 1986, *Bio*/*Techniques* 4:214; U.S. Patent 5,225,539; Jones *et al.,* 1986, *Nature* **321**:552-525; Verhoeyan *et al.* (1988) *Science* **239**:1534; and Beidler *et al.,* 1988, *J. Immunol.* **141**:4053-4060.

Completely human antibodies are particularly desirable for therapeutic treatment of human patients. Such antibodies can be produced using transgenic mice which are incapable of expressing endogenous immunoglobulin heavy and light chain genes, but which can express human heavy and light chain genes. The transgenic mice are immunised in the normal fashion with a selected antigen, e.g., all or a portion of a polypeptide used in the invention. Monoclonal antibodies directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg & Huszar (1995), *Int. Rev. Immunol*. **13**:65-93. For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognise a selected epitope can be generated using a technique referred to as "guided selection." In this approach a selected non-human monoclonal antibody, e.g., a mouse antibody, is used to guide the selection of a completely human antibody recognising the same epitope. (Jespers *et al.* (1994) *Bio*/*technology* **12**:899-903).

The antibodies used in the present invention can also be generated using various phage display methods known in the art. In phage display methods, functional antibody domains are displayed on the surface of phage particles which carry the polynucleotide sequences encoding them. In a particular, such phage can be utilised to display antigen binding domains expressed from a repertoire or combinatorial antibody library (e.g., human or murine). Phage expressing an antigen binding domain that binds the antigen of interest can be selected or identified with antigen, e.g., using labelled antigen or antigen bound or captured to a solid surface or bead. Phage used in these methods are typically filamentous phage including fd and M13 binding domains expressed from phage with Fab, Fv or disulphide stabilised Fv antibody domains recombinantly fused to either the phage gene III or gene VIII protein: Phage display methods that can be used to make the antibodies used in the present invention include those disclosed in Brinkman *et al*., *J. Immunol. Methods* **182:** 41-50 (1995); Ames *et al., J. Immunol. Methods* **184**:177-186 (1995); Kettleborough *et al., Eur. J. Immunol*. **24**:952-958 (1994); Persic *et al*., *Gene* **187** 9-18 (1997); Burton *et al*., *Advances in Immunology* **57**:191-280 (1994);. PCT/GB91/01134; WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in WO 92/22324; Mullinax *et al*., *BioTechniques* **12**(6):864-869 (1992); and Sawai *et al*., *AJRI* **34**:26-34 (1995); and Better *et al*., *Science* **240**:1041-1043 (1988).

Examples of techniques which can be used to produce single-chain Fvs and antibodies include those described in U.S. Patents 4,946,778 and 5,258,498; Huston *et al*., *Methods in Enzymology* **203**:46-88 (1991); Shu *et al., PNAS* **90**:7995-7999 (1993); and Skerra *et al*., *Science* **240**:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein *et al*., 1983, *Nature* **305**:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker *et al*., 1991, *EMBO J.* **10**:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details for generating bispecific antibodies see, for example, Suresh *et al*., *Methods in Enzymology,* 1986,**121**:210.

The invention provides for the use of functionally-active fragments, derivatives or analogues of the anti-polypeptide immunoglobulin molecules. "Functionally-active" means that the fragment, derivative or analogue is able to elicit anti-anti-idiotype antibodies (i.e., tertiary antibodies) that recognise the same antigen that is recognised by the antibody from which the fragment, derivative or analogue is derived. Specifically, in a preferred embodiment, the antigenicity of the idiotype of the immunoglobulin molecule may be enhanced by deletion of framework and CDR sequences that are C-terminal to the CDR sequence that specifically recognises the antigen. To determine which CDR sequences bind the antigen, synthetic peptides containing the CDR sequences can be used in binding assays with the antigen by any binding assay method known in the art.

The present invention provides antibody fragments such as, but not limited to, F(ab')2 fragments and Fab fragments. Antibody fragments which recognise specific epitopes may be generated by known techniques. F(ab')2 fragments consist of the variable region, the light chain constant region and the CH1 domain of the heavy chain and are generated by pepsin digestion of the antibody molecule. Fab fragments are generated by reducing the disulphide bridges of the F(ab')2 fragments. The invention also provides heavy chain and light chain dimmers of the antibodies of the invention, or any minimal fragment thereof such as Fvs or single chain antibodies (SCAs) (e.g., as described in U.S. Patent 4,946,778; Bird, 1988, *Science* **242**:423-42; Huston *et al.,* 1988, *Proc. Natl. Acad. Sci. USA* **85**:5879-5883; and Ward *et al*., 1989, *Nature* **334**:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in E. coli may be used (Skerra *et al*., 1988, *Science* **242**:1038-1041).

In other embodiments, the invention provides fusion proteins of the immunoglobulins of the invention (or functionally active fragments thereof), for example in which the immunoglobulin is fused via a covalent bond (e.g., a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo*, and enhance the delivery of an antigen across an epithelial barrier to the immune system.

The immunoglobulins used in the invention include analogues and derivatives that are either modified, i.e., by the covalent attachment of any type of molecule as long as such covalent attachment that does not impair immunospecific binding. For example, but not by way of limitation, the derivatives and analogues of the immunoglobulins include those that have been further modified, e.g., by glycosylation, acetylation, pegylation, phosphylation, amidation, derivatisation by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analogue or derivative may contain one or more non-classical amino acids.

The foregoing antibodies can be used in methods known in the art relating to the localisation and activity of the polypeptides used in the invention, e.g., for imaging or radioimaging these proteins, measuring levels thereof in appropriate physiological samples, in diagnostic methods, etc. and for radiotherapy.

The antibodies of the invention can be produced by any method known in the art for the synthesis of antibodies, in particular, by chemical synthesis or by recombinant expression, and are preferably produced by recombinant expression technique.

Recombinant expression of antibodies, or fragments, derivatives or analogues thereof, requires construction of a nucleic acid that encodes the antibody. If the nucleotide sequence of the antibody is known, a nucleic acid encoding the antibody may be assembled from chemically synthesised oligonudeotides (e.g., as described in Kutmeier *et al*., 1994, *BioTechniques* **17**:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g., an antibody cDNA library, or cDNA library generated from any tissue or cells expressing the antibody) by PCR amplification using synthetic primers hybridisable to the 3' and 5' ends of the sequence or by cloning using an oligonucleotide probe specific for the particular gene sequence.

If an antibody molecule that specifically recognises a particular antigen is not available (or a source for a cDNA library for cloning a nucleic acid encoding such an antibody), antibodies specific for a particular antigen may be generated by any method known in the art, for example, by immunising an animal, such as a rabbit, to generate polyclonal antibodies or, more preferably, by generating monoclonal antibodies. Alternatively, a clone encoding at least the Fab portion of the antibody may be obtained by screening Fab expression libraries (e.g., as described in Huse *et al.,* 1989, *Science* **246**:1275-1281) for clones of Fab fragments that bind the specific antigen or by screening antibody libraries (*See*, e.g., Clackson *et al.,* 1991, *Nature* **352**:624; Hane *et al.,* 1997 *Proc. Natl. Acad. Sci. USA* **94**:4937).

Once a nucleic acid encoding at least the variable domain of the antibody molecule is obtained, it may be introduced into a vector containing the nucleotide sequence encoding the constant region of the antibody molecule (see, e.g., WO 86/05807; WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulphide bond with an amino acid residue that does not contain a sulphydryl group. Such modifications can be carried out by any method known in the art for the introduction of specific mutations or deletions in a nucleotide sequence, for example, but not limited to, chemical mutagenesis, *in vitro* site directed mutagenesis (Hutchinson *et al*., 1978, *J. Biol. Chem*. **253**:6551), PCR based methods, etc.

In addition, techniques developed for the production of "chimeric antibodies" (Morrison *et al*., 1984, *Proc. Natl. Acad. Sci.* **81**:851-855; Neuberger *et al*., 1984, *Nature* **312**:604-608; Takeda *et al*., 1985, *Nature* **314**:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, e.g., humanised antibodies.

Once a nucleic acid encoding an antibody molecule has been obtained, the vector for the production of the antibody molecule may be produced by recombinant DNA technology using techniques well known in the art. Thus, methods for preparing the polypeptides used in the invention by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook *et al*. (1990, Molecular Cloning, A Laboratory Manual, 2d Ed., Cold Spring Harbor Laboratory, Cold Spring Harbor, NY) and Ausubel *et al*. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking *et al*., 198, *Gene* **45**:101; Cockett *et al*., 1990, *Bio*/*Technology* **8**:2).

A variety of host-expression vector systems may be utilised to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ*. These include but are not limited to microorganisms such as bacteria (e.g., *E. coli, B. subtilis*) transformed with recombinant bacteriophage DNA, plasmid DNA or cosmid DNA expression vectors containing antibody coding sequences; yeast (e.g., *Saccharomyces, Pichia*) transformed with recombinant yeast expression vectors containing antibody coding sequences; insect cell systems infected with recombinant virus expression vectors (e.g., baculovirus) containing the antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harbouring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther *et al.,* 1983, *EMBO J.* **2**:1791), in which the antibody coding sequence may be ligated individually into the vector in frame with the lac Z coding region so that a fusion protein is produced; pIN vectors (Inouye & Inouye, 1985, *Nucleic Acids Res.* **13**:3101-3109; Van Heeke & Schuster, 1989, *J. Biol. Chem.* **24**:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter). In mammalian host cells, a number of viral-based expression systems (e.g., an adenovirus expression system) may be utilised.

As discussed above, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein.

For long-term, high-yield production of recombinant antibodies, stable expression is preferred. For example, cells lines that stably express an antibody of interest can be produced by transfecting the cells with an expression vector comprising the nucleotide sequence of the antibody and the nucleotide sequence of a selectable (e.g., neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse *et al.,* 1983, *Mol. Cell. Biol.* **3**:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, *Nature* **322**:52; Kohler, 1980, *Proc. Natl. Acad. Sci. USA* **77**:2197). The coding sequences for the heavy and light chains may comprise cDNA or genomic DNA.

Once the antibody molecule used in the invention has been recombinantly expressed, it may be purified by any method known in the art for purification of an antibody molecule, for example, by chromatography (e.g., ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilising an antibody specific for the fusion protein being expressed. For example, a system described by Janknecht *et al.* allows for the ready purification of non-denatured fusion proteins expressed in human cell lines (Janknecht *et al.,* 1991, *Proc. Natl. Acad. Sci. USA* **88**:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

In a preferred embodiment, antibodies of the invention or fragments thereof are conjugated to a diagnostic or therapeutic moiety. The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

Antibodies used in the invention or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, e.g., Amon *et al*., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom *et al*., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications; Pinchera *et al*. (eds.), pp. 475-506 (1985); "Analysis, Results, And Future Prospective Of The Therapeutic Use Of Radiolabelled Antibody In Cancer Therapy", in Monoclonal Antibodies For Cancer Detection And Therapy, Baldwin et al. (eds.), pp. 303-16 (Academic Press 1985), and Thorpe *et al*., "The Preparation And Cytotoxic Properties Of Antibody-Toxin Conjugates", *Immunol. Rev.,* **62**:119-58 (1982).

Alternatively, an antibody can be conjugated to a second antibody to form an antibody heteroconjugate as described in U.S. Patent No. 4,676,980.

An antibody with or without a therapeutic moiety conjugated to it can be used as a therapeutic that is administered alone or in combination with cytotoxic factor(s) and/or cytokine(s).

As discussed herein, certain polypeptides, nucleic acid molecules and antibodies find use in the treatment or prophylaxis of breast cancer. Pharmaceutical formulations may be formulated, comprising at least one polypeptide or fragment thereof, nucleic acid molecule or antibody of use in the invention, optionally together with one or more pharmaceutically acceptable excipients, carriers or diluents. Preferably, the pharmaceutical formulation is for use as a vaccine and so any additional components will be acceptable for vaccine use. In addition, the skilled person will appreciate that one or more suitable adjuvants may be added to such vaccine preparations.

The medicament will usually be supplied as part of a sterile, pharmaceutical composition which will normally include a pharmaceutically acceptable carrier. This pharmaceutical composition may be in any suitable form (depending upon the desired method of administering it to a patient).

It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions).

Suitable excipients for tablets or hard gelatine capsules include lactose, maize starch or derivatives thereof, stearic acid or salts thereof.

Suitable excipients for use with soft gelatine capsules include for example vegetable oils, waxes, fats, semi-solid, or liquid polyols etc.

For the preparation of solutions and syrups, excipients which may be used include for example water, polyols and sugars. For the preparation of suspensions, oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in *Pharmaceutical Research,* **3(6)**:318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulisers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilised) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the polypeptide, nucleic acid or antibody used in of the present invention can vary between wide limits, depending upon the disease or disorder to be treated, the age and condition of the individual to be treated, etc. and a physician will ultimately determine appropriate dosages to be used. This dosage may be repeated as often as appropriate. If side effects develop the amount and/or frequency of the dosage can be reduced, in accordance with normal clinical practice.

In view of the importance of BCMP 7 in breast cancer, the following form additional aspects of the present invention:
i) a method of screening for compounds that modulate, i.e. up-regulate or down-regulate, the expression of a polypeptide used in the invention, which comprises the step of determining the presence or absence and/or quantifying at least one polypeptide or antibody of the invention in a biological sample;
ii) a method for monitoring/assessing breast cancer treatment in a patient, which comprises the step of determining the presence or absence and/or quantifying at least one polypeptide used in the invention in a biological sample obtained from said patient;
iii) method for the identification of metastatic breast cancer cells in a biological sample obtained from a subject, which comprises the step of determining the presence or absence and/or quantifying at least one polypeptide of the invention;
(iv) methods of treating breast cancer, comprising administering to a patient a therapeutically effective amount of a compound that modulates (e.g., upregulates or downregulates) or complements the expression or the biological activity (or both) of a polypeptide as defined herein in patients having breast cancer, in order to (a) prevent the onset or development of breast cancer; (b) prevent the progression of breast cancer; or (c) ameliorate the symptoms of breast cancer.

In the context of the present invention, the biological sample can be obtained from any source, such as a serum sample or a tissue sample, e.g. breast tissue. When looking for evidence of metastasis, one would look at major sites of breast metastasis such as lymph nodes, liver, lung and/or bone.

In one embodiment, breast tissue from a subject is analysed for quantitative detection of a polypeptide as defined herein, wherein a change in abundance of the polypeptide in the breast tissue from the subject relative to breast tissue from a subject or subjects free from breast cancer (*e*.*g*., a control sample or a previously determined reference range) indicates the presence of breast cancer.

The invention also provides diagnostic kits, comprising an antibody against a polypeptide as defined herein. In addition, such a kit may optionally comprise one or more of the following: (1) instructions for using the antibody for diagnosis, prognosis, therapeutic monitoring or any combination of these applications; (2) a labelled binding partner to the antibody; (3) a solid phase (such as a reagent strip) upon which the antibody is immobilised; and (4) a label or insert indicating regulatory approval for diagnostic, prognostic or therapeutic use or any combination thereof. If no labelled binding partner to the antibody is provided, the anti-polypeptide antibody itself can be labelled with a detectable marker, *e.g.,* a chemiluminescent, enzymatic, fluorescent, or radioactive moiety.

The invention also provides a kit comprising a nucleic acid probe capable of hybridising to RNA encoding a polypeptide as defined herein. In a specific embodiment, a kit comprises in one or more containers a pair of primers (*e*.*g*., each in the size range of 6-30 nucleotides, more preferably 10-30 nucleotides and still more preferably 10-20 nucleotides) that under appropriate reaction conditions can prime amplification of at least a portion of a nucleic acid encoding a polypeptide as defined herein, such as by polymerase chain reaction (see *e.g.,* Innis *et al*., 1990, PCR Protocols, Academic Press, Inc., San Diego, CA), ligase chain reaction (see EP 320,308) use of Qβ replicase, cyclic probe reaction, or other methods known in the art.

The invention provides methods for identifying agents, candidate compounds or test compounds that bind to a polypeptide as defined herein or have a stimulatory or inhibitory effect on the expression or activity of a polypeptide as defined herein.

The present invention also provides assays for use in drug discovery in order to identify or verify the efficacy of compounds for treatment or prevention of breast cancer. Test compounds can be assayed for their ability to modulate levels of a polypeptide as defined herein in a subject having breast cancer. Compounds able to modulate levels of a polypeptide as defined herein in a subject having breast cancer towards levels found in subjects free from breast cancer or to produce similar changes in experimental animal models of breast cancer can be used as lead compounds for further drug discovery, or used therapeutically. Expression of a polypeptide as defined herein can be assayed by, for example, immunoassays, gel electrophoresis followed by visualisation, detection of activity, or any other method taught herein or known to those skilled in the art. Such assays can be used to screen candidate drugs, in clinical monitoring or in drug development, where abundance of a polypeptide as defined herein can serve as a surrogate marker for clinical disease.

This invention further provides novel agents identified by the above-described screening assays and uses thereof for treatments as described herein. In addition, the invention also provides the use of an agent which interacts with, or modulates the activity of a polypeptide as defined herein in the manufacture of a medicament for the treatment of breast cancer.

The invention provides for treatment or prevention of various diseases and disorders by administration of a therapeutic compound. Such compounds include but are not limited to: a polypeptide as defined herein and analogues and derivatives (including fragments) thereof; antibodies thereto; nucleic acids encoding a polypeptide as defined herein, analogues, or derivatives; antisense nucleic acids to a gene encoding a polypeptide as defined herein, and agonists and antagonists of a gene encoding a polypeptide as defined herein or agonists and antagonists of a polypeptide as defined herein. An important feature of the present invention is the identification of a gene encoding a polypeptide as defined herein involved in breast cancer. Breast cancer can be treated or prevented by administration of a therapeutic compound that modulates function or expression of a polypeptide as defined herein in the breast tissue of breast cancer patients.

In one embodiment, one or more antibodies each specifically binding to a polypeptide as defined herein are administered alone or in combination with one or more additional therapeutic compounds or treatments. Examples of such treatments include, but are not limited to, taxol, cyclophosphamide, tamoxifen, and doxorubacin.

The compounds of the invention include but are not limited to any compound, *e.g*., a small organic molecule, protein, peptide, antibody, nucleic acid, etc. that restores the profile towards normal with the proviso that such compounds or treatments include, but are not limited to, taxol, cyclophosphamide, tamoxifen, and doxorubacin.

In another embodiment, symptoms of breast cancer may be ameliorated by decreasing the level or activity of a polypeptide as defined herein by using gene sequences encoding a polypeptide as defined herein in conjunction with well-known gene "knock-out," ribozyme or triple helix methods to decrease gene expression of the polypeptide. In this approach, ribozyme or triple helix molecules are used to modulate the activity, expression or synthesis of the gene, and thus to ameliorate the symptoms of breast cancer. Such molecules may be designed to reduce or inhibit expression of a mutant or non-mutant target gene. Techniques for the production and use of such molecules are well known to those of skill in the art.

Endogenous polypeptide expression can also be reduced by inactivating or "knocking out" the gene encoding the polypeptide, or the promoter of such a gene, using targetted homologous recombination (*e.g.,* see Smithies, *et al.,* 1985, *Nature* **317**:230-234; Thomas & Capecchi, 1987, *Cell* **51**:503-512; Thompson *et al*., 1989, *Cell* **5**:313-321; and Zijlstra *et al*., 1989, *Nature* **342**:435-438). For example, a mutant gene encoding a non-functional polypeptide (or a completely unrelated DNA sequence) flanked by DNA homologous to the endogenous gene (either the coding regions or regulatory regions of the gene encoding the polypeptide).can be used, with or without a selectable marker and/or a negative selectable marker, to transfect cells that express the target gene *in vivo.* Insertion of the DNA construct, via targeted homologous recombination, results in inactivation of the target gene. Such approaches are particularly suited in the agricultural field where modifications to ES (embryonic stem) cells can be used to generate animal offspring with an inactive target gene (*e*.*g*., see Thomas & Capecchi, 1987 and Thompson, 1989, *supra*). However this approach can be adapted for use in humans provided the recombinant DNA constructs are directly administered or targeted to the required site *in vivo* using appropriate viral vectors.

BCMP 7 has also been found to be highly expressed in prostate cancer cell lines. Accordingly, for the applications discussed above in respect of breast cancer, BCMP 7 finds the same application in respect of prostate cancer.

Preferred features of each aspect of the invention are as for each of the other aspects *mutatis mutandis*. The prior art documents mentioned herein are incorporated to the fullest extent permitted by law.

### Examples

The invention will now be described with reference to the following examples, which should not in any way be construed as limiting the scope of the present invention. The examples refer to the figures in which:
Figure 1 shows the nucleotide and predicted amino acid sequences of BCMP 7. The predicted N-terminal signal sequence is underlined. Mass spectra assigned to the predicted protein are in bold and underlined. Tandem mass spectra are in bold and italicised; and
Figure 2 shows tissue distribution of BCMP 7 mRNA. Levels of mRNA in normal tissues and breast and prostate carcinoma cell lines were quantified by real time RT-PCR. mRNA levels are expressed as the number of copies ng⁻¹ cDNA.
Figure 3 shows the expression of BCMP 7 in matched normal and tumour breast tissues. Levels of BCMP 7 mRNA in matched normal and tumour tissues from seven breast cancer patients were measured by real time RT-PCR. mRNA levels are expressed as the number of copies ng⁻¹ cDNA.

### Example 1: Identification and cloning of BCMP 7

The breast carcinoma cell line T-47D was cultured in DMF12 media, supplemented with 10% foetal calf serum, 2mM glutamine, 1% penicillin and 1% streptomycin. The cells were grown at 37°C in a humidified atmosphere of 95% air and 5% carbon dioxide.

10⁸ cells were harvested by trypsinisation and centrifugation, and used to prepare membrane proteins for separation by 1D PAGE (Bennett, J.P. Techniques in lipid and membrane biochemistry. Holland: Elsevier. (1982); Fujiki, Y., Fowler, S., Shio, H., Hubbard A.L. & Lazarow, P.B. Polypeptide and phospholipid composition of the membrane of rat liver peroxisomes: comparison with endoplasmic reticulum and mitochondrial membranes. *J. Cell Biol*. **93**, 103-110 (1982).). Following sonication of the harvested cells (MSE Soniprep 150, flat bottomed probe for 10 seconds at an amplitude of 5 microns), the cell homogenate was centrifuged at 4°C and 1000 x g for 10 min. Cell membranes were pelleted by centrifuging the supernatant at 4°C and 100,000 x g for 1 h, and the pellet washed by centrifugation in 1M NaCl.

The membrane protein was solubilized by homogenisation in Tx114 detergent (50mM Tris HCl, 0.2mM EDTA, 1.5% Tx114) (pH 7.4), and the protein mixture centrifuged at 13,000 x g for 3 min, followed by extraction of the soluble fraction with a mixture of methanol and chloroform (Boyd, R.S., Duggan, M.J., Shone, C.C. & Foster, K.A. The effect of botulinum neurotoxins on the release of insulin from the insulinoma cell lines HIT-15 and RINm5F. *J. Biol. Chem*. **270**,18216-18218 (1995)). The extracted protein sample was finally solubilized in 1D lysis buffer and the proteins separated by 1D PAGE.

### Mass Spectrometry

Proteins excised from the 1D gel were digested with trypsin and analysed by MALDI-TOF-MS (Voyager STR, Applied Biosystems) using a 337 nm wavelength laser for desorption and the reflectron mode of analysis. Two selected mass for BCMP 7 ([M+H] = 1293.7 and 1268.6) were further characterised by tandem mass spectrometry using a QTOF-MS equipped with a nanospray ion source, (Micromass UK Ltd.). Prior to MALDI analysis the samples were desalted and, concentrated using C18 Zip TipsTM (Millipore). Samples for tandem MS were purified using a nano LC system (LC Packings) incorporating C18 SPE material.

The uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was a database constructed of protein entries in the non-redundant database held by the National Centre for Biotechnology Information (NCBI) which is accessible at http://www.ncbi.nlm.nih.gov/.

Three spectra from protein BCMP 7 (two tandem spectra, Table 1, and one MALDI-mass spectra, Fig 1), were found to match the following mRNA records: accession number AF007791, AF038451 at http://www.ncbi.nlm.nih.gov/entrez/, defining an open reading frame (ORF) of 175 amino acids (Fig. 1).

**Table 1.**

| Amino Acid Sequence Information Derived from Tandem Mass Spectrometry Analysis of BCMP 7 | |
|---|---|
| Peptide Precursor Ion m/z | Sequence |
| 1293.7 | IMFVDPSLTVR |
| 1268.6 | HLSPDGQYVPR |

### A full length clone was amplified by PCR from Colon cDNA (Fig 1).

### Preparation of total RNA and cDNA synthesis

Total RNA was prepared from cultured cells and tissue samples using Trizol reagent (Life Technologies), according to the manufacturer's instructions, and resuspended in RNAse-free water at a concentration of 1µg/µl. 1 to 5µg total RNA were used as a template for cDNA synthesis using an oligo dT primer and the Superscript II reverse transcription kit (Life Technologies), cDNAs were column purified (Qiagen) and eluted at a concentration of 10ng/µl.

### Cloning of BCMP 7 cDNA

The predicted full length BCMP 7 ORF was amplified by PCR from colon cDNAs, using the following primers: F, 5' **CATCTCGAG**CAGAGTTGCCATGGAGAAAAT 3'; R, 5' **CATGGATCC**TTTCTTTACAATTCAGTCTTCAGC 3' (restriction sites in bold - not homologous to BCMP7 sequences). Reactions contained 10ng cDNA and reagents for PCR (Qiagen), and used the following cycling parameters: 40 cycles of 94°C for 30 seconds, 50°C for 30 seconds, 72°C for 30 seconds. The PCR products were column purified (Qiagen), cloned into a T/A vector (Invitrogen) and the nucleotide sequence subsequently verified (University of Oxford, Sequencing Facility, UK).

The predicted BCMP 7 protein is 100 % identical to hAG-2, a novel human protein encoded by a cDNA cloned from the MCF-7 breast cancer cell line (Thompson, D.A. & Weigel, R.J. hAG-2, the human homologue of the Xenopus laevis cement gland gene XAG-2, is co-expressed with estrogen receptor in breast cancer cell lines. *Biochem. Biophys. Res. Commun.* **251**, 111-116 (1998)). hAG-2 is the human homologue of XAG-2, a *Xenopus laevis* protein that is expressed in the cement gland during frog development (Aberger, F., Weidinger, G., Grunz, H. & Richter, K. Anterior specification of embryonic ectoderm: the role of the Xenopus cement gland-specific gene XAG-2. *Mech. Dev.* **72,** 115-130 (1998)).

BCMP 7 is predicted to be an extracellular protein with an N-terminal signal sequence (http://psort.nibb.ac.ip) (Figure 1).

### Example 2: Expression of BCMP 7 mRNA in human tissues

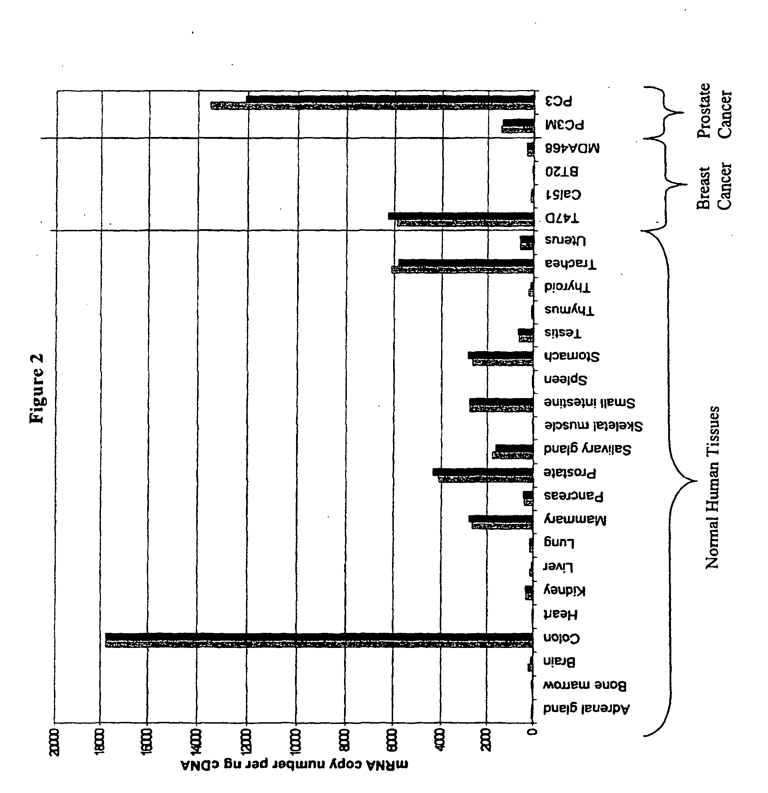

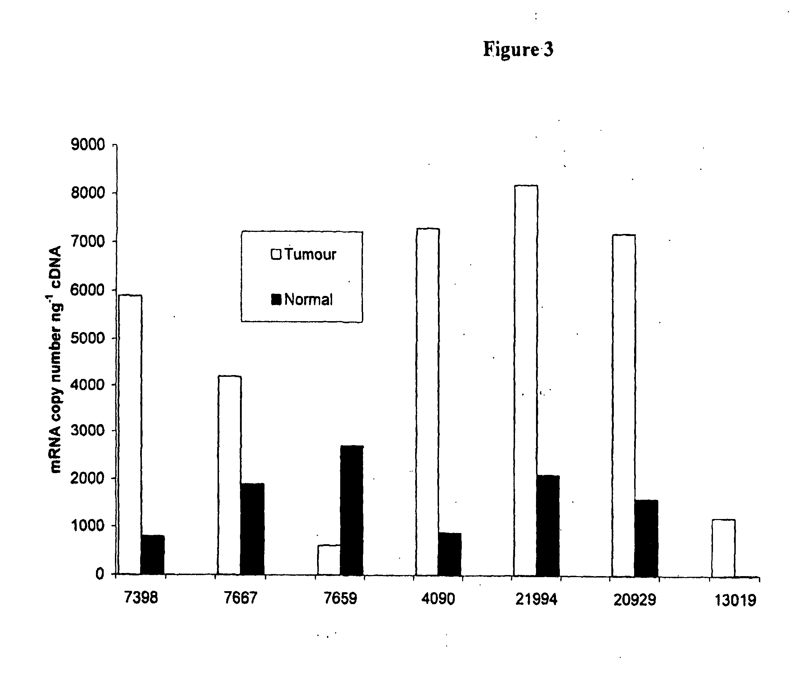

We used real time quantitative RT-PCR (Heid, C.A., Stevens, J., Livak, K.J. & Williams, P.M. Real time quantitative PCR. Genome Res. 6, 986-994 (1996); Morrison, T.B., Weis, J.J. & Wittwer, C.T. Quantification of low-copy transcripts by continuous SYBR Green I monitoring during amplification. Biotechniques 24, 954-958 (1998)) to analyse the distribution of BCMP 7 mRNA in normal human tissue mRNAs (Clontech) and breast and prostate cancer cell lines (Fig 2), and normal breast tissue derived from cosmetic reduction manunoplasties and cancerous breast tissue from surgery (Fig 3). Ethical approval for the normal and cancer breast samples was obtained at surgery (Oxford, UK). The primers used for PCR were as follows: sense, 5' AGATACCACAGTCAAACCTG 3', antisense, 5' GCACTCATCCAAGTGATGAA 3'. Reactions containing 10ng cDNA, prepared as described above, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 60°C for 1min. The accumulation ofPCR product was measured in real time as the increase in SYBR green fluorescence, and the data were analysed using the Sequence Detector program v1.6.3 (PE Biosystems). Standard curves relating initial template copy number to fluorescence and amplification cycle were generated using the amplified PCR product as a template, and were used to calculate BCMP 7 copy number in each sample.

The highest levels of BCMP 7 expression were observed in colon, with lower levels of expression in mammary gland, prostate, salivary gland, small intestine, stomach and trachea (Fig. 2). BCMP 7 mRNA was also detected in T-47D cells, the source of BCMP 7 protein used in this study, and expression in this cell line was elevated in comparison to normal mammary tissue. BCMP 7 mRNA was also detected in the prostate cancer cell lines PC3 and PC3M: expression in PC3 cells was elevated in comparison to normal prostate. Little or no BCMP 7 mRNA was detected in the other cell lines or normal tissues examined.

To examine whether the observed elevation in BCMP 7 expression in some breast carcinoma lines is reiterated in clinical samples, we also measured the expression of mRNA in matched normal and tumour tissue samples from seven breast cancer patients (Fig 3). BCMP 7 expression was increased in six of the seven tumour samples, relative to their matched normal tissues, with four of the samples showing more than 4-fold elevation in expression. These observations suggest that this protein has potential as a therapeutic target.

hAG-2 expression was shown to be coincident with expression of the ER, and hAG-2 mRNA levels in MCF7 cells increased when the cells were treated with estradiol (Thompson and Weigel, *supra*). In agreement with these data, BCMP 7 mRNA was detected in ER positive T-47D cells, but no expression was observed in the ER negative cell lines CAL51, BT20 and MDA-MB-468 (Figure 2).

### Chromosomal localisation

A Blast search of a human genomic database with the BCMP 7 cDNA sequence (Fig. 1) found that Genbarik entry AC073333 contains the entire BCMP7 gene. AC073333 contains sequences from human chromosome 7. The BCMP 7 gene has been further localized to chr7p21.3 (Petek, E., Windpassinger, C., Egger, H., Kroisel, P.M. & Wagner, K. Localization of the human anterior gradient-2 gene (AGR2) to chromsome 7p21.3 by radiation hybrid mapping and fluorescence in situ hybridisation. *Cytogenet. Cell Genet.* **89,** 141-142 (2000)).

## Claims

1. A method of screening for and/or diagnosis of breast cancer in a subject, which method comprises the step of detecting and/or quantifying the amount of a polypeptide in a biological sample obtained from said subject, wherein the polypeptide:
a) comprises or consists of the amino acid sequence shown in Figure 1 (SEQ ID NO: 8);
b) is a derivative having one or more amino acid substitutions, deletions or insertions relative to the amino acid sequence shown in Figure 1 (SEQ ID NO: 8); or
c) is a fragment of a polypeptide as defined in a) or b) above, which is at least ten amino acids long.

2. The method according to claim 1 wherein the polypeptide:
a) consists of the amino acid sequence shown in Figure 1 (SEQ ID NO: 8); or
b) is a derivative having a sequence identity of at least 90% of the amino acid sequence shown in Figure 1 (SEQ ID NO: 8).

3. The use of at least one polypeptide as defined in claim 1 or 2 in the preparation of a medicament for use in the prophylaxis and/or treatment of breast cancer.

4. The use as according to claim 3, wherein the medicament is a vaccine.

5. A method of screening for and/or diagnosis of breast cancer in a subject, which method comprises the step of detecting and/or quantifying the amount of a nucleic acid in a biological sample obtained from said subject, wherein the nucleic acid molecule:
a) comprises or consists of the DNA sequence shown in Figure 1 (SEQ ID NO: 7) or its RNA equivalent;
b) has a sequence which is complementary to the sequences of a);
c) has a sequence which codes for the same polypeptide as the sequences of a) or b);
d) has a sequence which shows substantial identity with any of those of a), b) and c); or
e) a sequence which codes for a derivative or fragment of an amino acid molecule shown in Figure 1 (SEQ ID NO: 8).

6. The use of at least one nucleic acid as defined in claim 5 in the preparation of a medicament for use in the prophylaxis and/or treatment of breast cancer.

7. The use of an antibody which binds to at least one polypeptide as claimed in claim 1 or 2 for screening for and/or diagnosis of breast cancer in a subject according to claim 1.

8. The use of an antibody which binds to at least one polypeptide as defined in claim 1 or 2 in the preparation of a medicament for use in the prophylaxis and/or treatment of breast cancer.

9. The use according to claim 7 or 8, wherein the antibody binds specifically to a polypeptide as defined in claim 1 or 2.

10. The use according to any one of claims 7 to 9, wherein the antibody is monoclonal.

11. The use according to any one of claims 8 to 10, wherein the antibody is conjugated to a therapeutic moiety.

12. The use according to claim 11, wherein the therapeutic moiety is selected from a second antibody or a fragment or derivative thereof, a cytotoxic agent or a cytokine.

13. A method of screening for anti-breast cancer compounds that modulate the expression of a polypeptide as defined in claim 1 or 2 which comprises the step of determining the presence or absence and/or quantifying at least one polypeptide as defined in claim 1 or 2 or at least one antibody as defined in claim 8 or 9 in a biological sample.

14. A method for monitoring/assessing breast cancer treatment in a patient, which comprises the step of determining the presence or absence and/or quantifying at least one polypeptide as defined in claim 1 or 2 or at least one antibody as defined in claim 8 or 9 in a biological sample obtained from said patient.

15. A method for the identification of metastatic breast cancer cells in a biological sample obtained from a subject, which comprises the step of determining the presence or absence and/or quantifying at least one polypeptide as defined in claim 1 or 2 or at least one antibody as defined in claim 8 or 9.

## Patentansprüche

1. Verfahren zum Screening nach und/oder zur Diagnose von Brustkrebs in einem Subjekt, das den Schritt des Detektierens und/oder des Quantifizierens der Menge eines Polypeptids in einer biologischen Probe umfasst, welche von dem Subjekt erhalten wurde, wobei das Polypeptid:
a) aus der in Figur 1 (SEQ ID NO:8) gezeigten Aminosäuresequenz besteht oder diese umfasst;
b) ein Derivat mit einer oder mehreren Aminosäure-Substitutionen, -Deletionen oder -Insertionen in Bezug auf die in Figur 1 (SEQ ID NO:8) gezeigte Aminosäuresequenz ist; oder
c) ein Fragment eines wie oben in a) oder b) definierten Polypeptids ist, das mindestens 10 Aminosäuren lang ist.

2. Verfahren nach Anspruch 1, bei dem das Polypeptid:
a) aus der in Figur 1 (SEQ ID NO:8) gezeigten Aminosäuresequenz besteht; oder
b) ein Derivat mit einer Aminosäuresequenzidentität von mindestens 90 % zu der in Figur 1 (SEQ ID NO:8) gezeigte Aminosäuresequenz ist.

3. Verwendung von mindestens einem wie in Anspruch 1 oder 2 definierten Polypeptid bei der Herstellung eines Medikamentes zur Verwendung bei der Prophylaxe und/oder bei der Behandlung von Brustkrebs.

4. Verwendung nach Anspruch 3, **dadurch gekennzeichnet, dass** das Medikament ein Impfstoff ist.

5. Verfahren zum Screening nach und/oder zur Diagnose von Brustkrebs in einem Subjekt, das den Schritt des Detektierens und/oder des Quantifizierens der Menge einer Nukleinsäure in einer biologischen Probe umfasst, welche von dem Subjekt erhalten wurde, und wobei das Nukleinsäure-Molekül:
a) aus der in Figur 1 (SEQ ID NO:7) gezeigten DNA-Sequenz oder ihrem RNA-Äquivalent besteht oder diese(s) umfasst oder;
b) eine Sequenz aufweist, die komplementär zu der Sequenz von a) ist;
c) eine Sequenz aufweist, die für das gleiche Polypeptid kodiert wie die Sequenzen in a) oder b);
d) eine Sequenz aufweist, die eine wesentliche Identität zu irgendeiner der in a), b) und c) dargestellten Sequenzen zeigt; oder
e) eine Sequenz aufweist, die für ein Derivat oder Fragment eines in Figur 1 (SEQ ID NO:8) gezeigten Aminosäure-Moleküls kodiert.

6. Verwendung von mindestens einer wie in Anspruch 5 definierten Nukleinsäure bei der Herstellung eines Medikamentes zur Verwendung bei der Prophylaxe und/oder der Behandlung von Brustkrebs.

7. Verwendung eines Antikörpers, der an mindestens ein wie in Anspruch 1 oder 2 definiertes Polypeptid bindet, zum Screening nach und/oder zur Diagnose von Brustkrebs in einem Subjekt nach Anspruch 1.

8. Verwendung eines Antikörpers, der an mindestens ein wie in Anspruch 1 oder 2 definiertes Polypeptid bindet, bei der Herstellung eines Medikamentes zur Verwendung bei der Prophylaxe und/oder bei der Behandlung von Brustkrebs.

9. Verwendung nach Anspruch 7 oder 8, **dadurch gekennzeichnet, dass** der Antikörper spezifisch an ein wie in Anspruch 1 oder 2 definiertes Polypeptid bindet.

10. Verwendung nach einem der Ansprüche 7 bis 9, **dadurch gekennzeichnet, dass** der Antikörper monoklonal ist.

11. Verwendung nach einem der Ansprüche 8 bis 10, **dadurch gekennzeichnet, dass** der Antikörper an einen therapeutischen Rest konjugiert ist.

12. Verwendung nach Anspruch 11, **dadurch gekennzeichnet, dass** der therapeutische Rest ausgewählt ist aus einem sekundären Antikörper oder einem Fragment oder einem Derivat desselben, einem zytotoxischen Mittel oder einem Zytokin.

13. Verfahren zum Screening nach Anti-Brustkrebs-Verbindungen, die die Expression eines wie in Anspruch 1 oder 2 definierten Polypeptids modulieren, wobei das Verfahren den Schritt der Bestimmung des Vorhandenseins oder Fehlens und/oder des Quantifizierens von mindestens einem wie in Anspruch 1 oder 2 definierten Polypeptid oder von mindestens einem wie in Anspruch 8 oder 9 definierten Antikörper in einer biologischen Probe umfasst.

14. Verfahren zum Verfolgen/Beurteilen der Brustkrebsbehandlung in einem Patienten, das den Schritt der Bestimmung des Vorhandenseins oder Fehlens und/oder des Quantifizierens von mindestens einem wie in Anspruch 1 oder 2 definierten Polypeptid oder von mindestens einem wie in Anspruch 8 oder 9 definierten Antikörper in einer biologischen Probe umfasst, welche von dem Patienten erhalten wurde.

15. Verfahren zur Identifizierung von metastatischen Brustkrebszellen in einer biologischen Probe, die von einem Subjekt erhalten wurde, wobei das Verfahren den Schritt der Bestimmung des Vorhandenseins oder Fehlens und/oder des Quantifizierens von mindestens einem wie in Anspruch 1 oder 2 definierten Polypeptid oder von mindestens einem wie in Anspruch 8 oder 9 definierten Antikörper umfasst.

## Revendications

1. Procédé de dépistage et/ou de diagnostic du cancer du sein chez un sujet, lequel procédé comprend l'étape de détection et/ou de quantification de la quantité d'un polypeptide présente dans un échantillon biologique provenant dudit sujet, dans lequel le polypeptide :
a) comprend ou consiste en la séquence d'acides aminés présentée dans la figure 1 (SEQ ID NO: 8) ;
b) est un dérivé ayant une ou plusieurs substitutions, délétions ou insertions d'acides aminés par rapport à la séquence d'acides aminés présentée dans la figure 1 (SEQ ID NO:8) ; ou
c) est un fragment d'un polypeptide tel que défini en a) ou b) ci-dessus, qui a une longueur d'au moins dix acides aminés.

2. Procédé selon la revendication 1, où le polypeptide :
a) consiste en la séquence d'acides aminés présentée dans la figure 1 (SEQ ID NO:8) ; ou
b) est un dérivé ayant une identité de séquence d'au moins 90% avec la séquence d'acides aminés présentée dans la figure 1 (SEQ ID NO:8).

3. Utilisation d'au moins un polypeptide selon la revendication 1 ou 2, dans la préparation d'un médicament utilisable dans la prophylaxie et/ou le traitement du cancer du sein.

4. Utilisation selon la revendication 3, dans laquelle le médicament est un vaccin.

5. Procédé de dépistage et/ou de diagnostic du cancer du sein chez un sujet, lequel procédé comprend l'étape de détection et/ou de quantification de la quantité d'un acide nucléique présente dans un échantillon biologique provenant dudit sujet, dans lequel la molécule d'acide nucléique :
a) comprend ou consiste en la séquence d'ADN présentée dans la figure 1 (SEQ ID NO: 7) ou son équivalent d'ARN ;
b) a une séquence qui est complémentaire des séquences de a) ;
c) a une séquence qui code pour le même polypeptide que les séquences de a) ou de b) ;
d) a une séquence qui présente une identité substantielle avec l'un quelconque de celles de a), b) et c) ; ou
e) une séquence qui code pour un dérivé ou fragment d'une molécule d'acides aminés présentée dans la figure 1 (SEQ ID NO: 8).

6. Utilisation d'au moins un acide nucléique selon la revendication 5, dans la préparation d'un médicament utilisable dans la prophylaxie et/ou le traitement du cancer du sein.

7. Utilisation d'un anticorps qui se lie à au moins un polypeptide selon la revendication 1 ou 2, pour le dépistage et/ou le diagnostic du cancer du sein chez un sujet selon la revendication 1.

8. Utilisation d'un anticorps qui se lie à au moins un polypeptide selon la revendication 1 ou 2, dans la préparation d'un médicament utilisable dans la prophylaxie et/ou le traitement du cancer du sein.

9. Utilisation selon la revendication 7 ou 8, dans laquelle l'anticorps se lie spécifiquement à un polypeptide selon la revendication 1 ou 2.

10. Utilisation selon l'une quelconque des revendications 7 à 9, dans laquelle l'anticorps est un anticorps monoclonal.

11. Utilisation selon l'une quelconque des revendications 8 à 10, dans laquelle l'anticorps est conjugué à une partie thérapeutique.

12. Utilisation selon la revendication 11, dans laquelle le groupe thérapeutique est choisie parmi un second anticorps ou un fragment ou dérivé de celui-ci, un agent cytotoxique ou une cytokine.

13. Procédé de criblage pour des composés anti-cancer du sein qui modulent l'expression d'un polypeptide selon les revendication 1 ou 2, qui comprend l'étape de détermination de la présence ou de l'absence et/ou de quantification d'au moins un polypeptide selon la revendication 1 ou 2, ou d'au moins un anticorps selon la revendication 8 ou 9, dans un échantillon biologique.

14. Procédé de suivi/évaluation du traitement du cancer du sein chez un patient, qui comprend l'étape de détermination de la présence ou de l'absence et/ou de quantification d'au moins un polypeptide selon la revendication 1 ou 2, ou d'au moins un anticorps selon la revendication 8 ou 9, dans un échantillon biologique obtenu à partir dudit patient.

15. Procédé pour l'identification de cellules de métastases d'un cancer du sein dans un échantillon biologique obtenu à partir d'un sujet, qui comprend l'étape de détermination de la présence ou de l'absence et/ou de quantification d'au moins un polypeptide selon la revendication 1 ou 2, ou d'au moins un anticorps selon la revendication 8 ou 9.
